# NOUVEAU FASCICULE DE BREVET EUROPEEN

(11) **EP 1 105 374 B2**
(45) Date de publication et mention de la décision concernant l'opposition: **12.09.2007**
(45) Mention de la délivrance du brevet: 04.06.2003
(21) Numéro de dépôt: 99931349.7
(22) Date de dépôt: 15.07.1999
(51) Int. Cl.: C07D 201/08

(54) **PROCEDE DE DESHYDRATATION DE LACTAME**
VERFAHREN ZUR LACTAMENTWÄSSERUNG
LACTAM DEHYDRATION METHOD

(30) Priorité: 28.07.1998 FR 9809808
(43) Date de publication de la demande: 13.06.2001
(73) Titulaire: RHODIA POLYAMIDE INTERMEDIATES, 69192 Saint-Fons (FR)
(72) Inventeur: BOCQUENET, Gérald, F-69360 Communay (FR); COURTEMANCHE, Yves, F-69003 Lyon (FR); HOUSSIER, Patrick, F-69310 Pierre-Bénite (FR)
(74) Mandataire: Esson, Jean-Pierre
(86) Numéro de dépôt international: PCT/FR1999/001732
(87) Numéro de publication internationale: WO 2000/006540

(56) Documents cités:
- EP-A- 0 659 741
- WO-A-95/14664
- WO-A-95/14665
- WO-A-96/17826
- WO-A-96/20923
- WO-A-96/22974
- WO-A-98/05636
- DE-A- 3 007 330
- DE-A- 3 825 524
- GB-A- 990 124
- US-A- 2 301 964
- US-A- 4 013 640
- US-A- 4 625 023
- US-A- 5 495 016
- US-A- 5 496 941
- US-A- 5 693 793
- Chemiker Kalender, 2.Aufl., 1974, 578-579,
- Grundlagen der chem. Technol., 2. Aufl., 1978, 461-464
- Lexikon Chemische Technik, 1. Aufl., 1988, 73-75
- Fundamental Aspects of Crystallization and Precipitation Processes, vol. 83, no. 253, 1987, 128, 143, 144, 155
- Ullmann's Encyclopedia of Ind. Chem., 5th ed., vol. B3, Unit Operation II, VCH Verlagsgesellschaft mbH, Weinheim (Germany) 1988, 4-56 - 4-59, 19-2 - 19-20
- CAV, juin 1973, 95-97
- Gerthsen-Kneser-Vogel, Physik, 13. Aufl., Springer-Verlag, 1977, 188
- C. Judson King, Separation Processes, 2nd ed., Mc-Graw-Hill Book Company, New York, 696, 699, 700

## Description

La présente invention concerne un procédé de séparation de l'eau d'une solution aqueuse de lactame.

Elle s'applique plus particulièrement à un mélange issu de la réaction entre un aminonitrile et l'eau (réaction aussi appelée hydrolyse cyclisante).

Lors de l'hydrolyse cyclisante d'un aminonitrile pour former un lactame, il y a également formation d'une molécule d'ammoniac par molécule de lactame. En fin de réaction, le mélange réactionnel contient donc au moins le lactame produit, l'eau en excès et l'ammoniac.

Il peut contenir des quantités moindres d'aminonitrile n'ayant pas réagi ou d'éventuels sous-produits de la réaction.

Il peut également comporter un solvant éventuellement mis en oeuvre dans la réaction d'hydrolyse cydisante.

L'hydrolyse cyclisante peut être réalisée en phase vapeur ou en phase liquide. Le procédé de l'invention peut s'appliquer aux mélanges issus de l'un ou l'autre de ces modes de préparation.

Pour la description des différents procédés de préparation de lactame par hydrolyse cyclisante d'un aminonitrile, on peut se référer par exemple au brevet EP-A-0 659 741 ou à la demande internationale de brevet WO-A-96/22974 ou encore aux demandes internationales de brevets WO-A-95/14664 et 95/14665 et US-A- 5693793.

Les aminonitriles utilisés pour préparer les lactames sont plus particulièrement les aminonitriles ayant de 4 à 12 atomes de carbone, préférentiellement les aminonitriles aliphatiques linéaires ou ramifiés.

A titre d'exemples de tels aminonitriles, on peut citer ceux qui proviennent de l'hydrogénation en fonction amine primaire d'une des deux fonctions nitriles des dinitriles tels que l'adiponitrile, le méthylglutaronitrile, l'éthylsuccinonitrile, le diméthylsuccinonitrile, le succinonitrile, le glutaronitrile, le dodecanedinitrile.

Parmi les solutions issues de l'hydrolyse cyclisante d'aminonitrile en lactame, celles qui correspondent à la préparation du caprolactame à partir d'amino-6 capronitrile et d'eau, sont les plus importantes au plan industriel, car ledit caprolactame conduit par polymérisation au polyamide 6.

GB-A-990124 décrit un procédé de purification du ε-caprolactame issu du réarrangement de Beckmann par distillation à un pH 3.2-4.2.

Le procédé de l'invention se rapportera donc plus particulièrement à la distillation de l'eau à partir de solutions aqueuses de caprolactame, mais cette distillation peut être transposée aux solutions aqueuses d'autres lactames.

Préalablement, il est nécessaire d'avoir séparé, généralement par distillation, l'ammoniac formé dans la réaction.

Un mode de séparation de l'eau qui peut être envisagé, consiste à distiller l'eau sous une pression réduite (inférieure ou égale à 20 mbar absolus par exemple), de manière à ne pas dépasser 145°C en pied de colonne.

Un tel procédé implique de condenser l'eau ainsi distillée, par une circulation d'un fluide (tel que l'eau) à une température inférieure ou égale à 15°C. A titre d'exemple, cela nécessite la présence d'un groupe frigorifique d'une puissance d'environ 1,2 mégawatt pour traiter de l'ordre de 8 tonnes/heure d'une solution aqueuse de lactame ayant une concentration pondérale en solutés de 60 % environ.

La présente invention a pour objet un procédé de séparation de l'eau (que l'on pourra également appeler déshydratation) d'une solution aqueuse de lactame, procédé qui est plus économique tant au plan de l'investissement nécessaire pour le mettre en oeuvre, qu'au plan de l'énergie consommée.

Elle consiste plus précisément en un procédé de séparation de l'eau d'une solution aqueuse de lactame, caractérisé en ce que :
- l'on distille l'eau dans une première colonne de distillation en maintenant une température en pied inférieure ou égale à 160°C et de préférence inférieure ou égale à 145°C, et sous une pression absolue de 50 millibars à 200 millibars, le distillat étant condensé à une température de 30°C à 60°C, avec un système fluide de refroidissement :
- le lactame restant en pied est ensuite soumis à une distillation dans une deuxième colonne de distillation en maintenant une température en pied inférieure ou égale à 160°C et de préférence inférieure ou égale à 145°C et sous une pression de 10 à 45 millibars, afin de séparer l'eau qu'il contient encore;
- l'eau, contenant des traces de lactame, distillée dans la deuxième colonne, est récupérée sous forme de vapeur à une température supérieure ou égale à 70°C et est recyclée dans la première colonne.

En opérant selon le procédé de l'invention, il n'est pas nécessaire d'avoir un groupe frigorifique pour condenser l'eau en tête de la première colonne.

L'eau, contenant des traces de lactame, distillée dans la deuxième colonne est recyclée dans la première colonne de distillation. Ce recyclage peut être réalisé après une condensation de l'eau à la pression de la deuxième colonne, soit entre 10 et 45 millibars absolus, correspondant à une température de 5°C à 30°C. Mais une telle variante ne nécessite qu'un groupe frigorifique de puissance environ 100 à 200 fois moindre que celle du groupe frigorifique qui aurait été nécessaire dans le cadre d'une distillation avec une colonne unique.

Selon un autre mode de réalisation de l'invention, l'eau contenant des traces de lactame, distillée dans la seconde colonne peut être comprimée à raide d'un éjecteur à vapeur jusqu'à une pression permettant de réaliser sa condensation à une température supérieure à 15°C, par un fluide de refroidissement usuel tel que l'air atmosphérique ou de l'eau à température ambiante. Dans ce cas la pression de refoulement des éjecteurs à vapeur doit être au plus égale à la pression de la première colonne, avantageusement comprise entre 25 millibars absolus et 50 à 200 millibars absolus, pression de la première colonne. Dans ce mode de réalisation, les groupes frigorifiques sont supprimés.

Globalement le présent procédé se caractérise, pour une capacité égale de distillation de solution aqueuse de lactame, par une consommation totale d'énergie beaucoup plus faible, pouvant se décomposer en une consommation d'électricité de 100 à 200 fois plus faible comme indiqué précédemment, et une consommation supplémentaire d'énergie sous forme de vapeur, pour chauffer la deuxième colonne, qui ne représente qu'environ moins de 15 % du gain réalisé en énergie sous forme d'électricité.

L'eau récupérée en tête de la première colonne est condensée à la pression de cette colonne, c'est à dire entre 50 millibars et 200 millibars correspondant à une température comprise entre 35 et 60 °C, compatible avec la plupart des fluides de refroidissement généralement utilisés, tels que l'air atmosphérique ou l'eau issue de rivières ou de tours de refroidissement.

L'eau récupérée en tête de la deuxième colonne peut être partiellement condensée à 70 °C environ avant d'être condensée totalement, puis de rejoindre la première colonne. Cette disposition permet de limiter la quantité d'eau à condenser dans des conditions particulières et donc de diminuer la quantité d'énergie nécessaire pour effectuer cette opération.

Avant le recyclage dans la première colonne de cette eau condensée, il peut être avantageux d'ajouter une certaine quantité d'eau pour diminuer la concentration en caprolactame et éviter sa précipitation.

En outre, malgré la nécessité d'une colonne supplémentaire, l'investissement nécessaire, à production égale, est également plus faible dans le procédé selon l'invention que dans un procédé réalisé avec une seule colonne, compte tenu de la puissance frigorifique considérable nécessaire dans ce dernier procédé.

La solution aqueuse de lactame, préférentiellement de caprolactame, dont l'ammoniac a été éliminé, a une concentration en lactame qui varie largement selon les conditions de préparation dudit lactame, notamment le rapport molaire initial eau/aminonitrile, la présence ou non d'un solvant organique. La concentration en lactame peut varier en général de 5 % à 80 % en poids par rapport au poids total de la solution et de préférence de 20 % à 75 %.

L'eau à distiller présentant des caractéristiques physiques très différentes du lactame dont elle doit être séparée, il n'est pas nécessaire d'utiliser des colonnes de distillation présentant un très grand nombre de plateaux théoriques.

Des colonnes, en particulier à garnissage, avec au moins deux plateaux théoriques conviennent très bien pour la mise en oeuvre du procédé de l'invention. Des colonnes ayant un nombre de plateaux théoriques de 2 à 10 peuvent être utilisées par exemple. Le garnissage utilisé pourra être du type garnissage vrac ou garnissage ordonné tel que proposé par les différents constructeurs et dimensionné selon les règles de l'art. Il est également possible d'utiliser des colonnes à plateaux de même efficacité, bien que cela soit moins intéressant du point de vue des pertes de charges.

Le procédé de l'invention n'exclut pas la possibilité de réaliser la séparation de l'eau de la solution aqueuse de lactame en utilisant plus de deux colonnes.

Mais, généralement, cela n'est pas utile et, en outre, les avantages économiques qu'apporte le procédé décrit précédemment seraient moindres si l'on augmentait de manière trop importante le nombre de colonnes à distiller. Les exemples qui suivent illustrent l'invention.

### EXEMPLE 1 distillation avec deux colonnes en série fonctionnant à des pressions différentes.

Distillation d'une solution aqueuse de caprolactame ayant la composition suivante :
- eau : 40 % en poids
- caprolactame : 60 % en poids

Les caractéristiques de la première colonne de distillation sont les suivantes :
- nombre d'étages de distillation (plateaux théoriques) : 4
- taux de reflux : 0,1
- pression opératoire absolue en tête : 0,13 bar
- température de tête : 50°C
- température de pied : 135°C
- charge thermique du bouilleur : 3,9 Gcal/h
- charge thermique du condenseur : 3,6 Gcal/h.

Les caractéristiques de la deuxième colonne de distillation sont les suivantes :
- nombre d'étages de distillation (plateaux théoriques) : 3
- pression opératoire absolue en tête : 0,025 bar
- température de tête : 130°C
- température de pied : 145°C
- charge thermique du bouilleur : 0,08 Gcal/h
- charge thermique du condenseur partiel tempéré à 70 °C : 0,02 Gcalh
- charge thermique du condenseur d'eau à recycler vers la première colonne : 0,02 Gcal/h
- puissance consommée du groupe frigorifique pour condenser l'eau en tête de la deuxième colonne : 10 kW, avec une condensation à 35°C.

Le caprolactame débarrassé de son eau qui a été obtenu dans cet exemple a la composition suivante :
- eau : 0,010 % en poids
- caprolactame : 99,990 % en poids

### ESSAI COMPARATIF : distillation classique avec groupe frigorifique en tête

Distillation d'un produit ayant la composition suivante :
- eau : 40 % en poids
- caprolactame : 60 % en poids.

Les caractéristiques de la colonne de distillation sont les suivantes:
- nombre d'étages de distillation : 4
- taux de reflux : 0,1
- pression opératoire absolue en tête : 0,015 bar
- température de tête : 13°C
- température de pied : 140°C
- charge thermique du bouilleur : 3,6 Gcal/h
- charge thermique du condenseur : 3,4 Gcal/h
- puissance consommée du groupe frigorifique : 1 200 kW, avec une condensation à 35°C.

Le caprolactame débarrassé de son eau qui a été obtenu dans l'essai comparatif a la composition suivante :
- eau : 0,004 % en poids
- caprolactame : 99,996 % en poids.

## Revendications

1. Procédé de séparation de l'eau d'une solution aqueuse de lactame, **caractérisé en ce que**:
- l'on distille l'eau dans une première colonne de distillation en maintenant une température en pied inférieure ou égale à 160°C et sous une pression absolue de 50 millibars à 200 millibars, le distillat étant condensé à une température de 30°C à 60°C avec un fluide de refroidissement,
- le lactame restant en pied est ensuite soumis à une distillation dans une deuxième colonne de distillation en maintenant une température en pied inférieure ou égale à 160°C et sous une pression de 10 millibars à 45 millibars
- l'eau, contenant des traces de lactame, distillée dans la deuxième colonne, est récupérée sous forme de vapeur à une température supérieure ou égale à 70°C et recyclée dans la première colonne.

2. Procédé selon la revendication 1, **caractérisé en ce que** l'on maintient en pied de la première colonne une température inférieure ou égale à 145°C.

3. Procédé selon l'une des revendications 1 ou 2, **caractérisé en ce que** l'on maintient en pied de la deuxième colonne une température inférieure ou égale à 145°C.

4. Procédé selon l'une des revendications 1 à 3, **caractérisé en ce que** le lactame est le caprolactame.

5. Procédé selon l'une des revendications 1 à 4, **caractérisé en ce que** le recyclage de l'eau est réalisé après une condensation à une température de 5°C à 30°C sous une pression de 10 à 45 millibars.

6. Procédé selon l'une des revendications 1 à 4, **caractérisé en ce que** le recyclage de l'eau contenant des traces de lactames est réalisé après compression de l'eau distillée à une pression au plus égale à la pression de première colonne et condensation à une température supérieure à 15°C.

7. Procédé selon l'une des revendications précédentes, **caractérisé en ce que** de l'eau est ajoutée dans l'eau contenant des traces de lactame, distillée dans la deuxième colonne avant recyclage dans la première colonne.

8. Procédé selon l'une des revendications 1 à 7, **caractérisé en ce que** la première colonne de distillation et la deuxième colonne de distillation ont chacune au moins deux plateaux théoriques et de préférence de 2 à 10 plateaux théoriques.

9. Procédé selon l'une des revendications 1 à 8, **caractérisé en ce que** les colonnes de distillation sont des colonnes à garnissage.

## Claims

1. Process for separating the water from an aqueous lactam solution, **characterized in that** :
- the water is distilled in a first distillation column by maintaining a temperature at the bottom of the column less than or equal to 160°C and at an absolute pressure of 50 millibars to 200 millibars, the distillate being condensed at a temperature of 30°C to 60°C using a coolant;
- the lactam remaining at the bottom of the column is then subjected to a distillation in a second distillation column by maintaining a temperature at the bottom of the column less than or equal to 160°C and at a pressure of 10 millibars to 45 millibars;
- the water, containing traces of lactam, distilled in the second column, is recovered in the form of vapour at a temperature greater than or equal to 70°C and recycled into the first column.

2. Process according to Claim 1, **characterized in that** a temperature less than or equal to 145°C is maintained at the bottom of the first column.

3. Process according to either of Claims 1 and 2, **characterized in that** a temperature less than or equal to 145°C is maintained at the bottom of the second column.

4. Process according to one of Claims 1 to 3, **characterized in that** the lactam is caprolactam.

5. Process according to one of Claims 1 to 4, **characterized in that** the water is recycled after condensing it at a temperature of 5°C to 30°C at a pressure of 10 to 45 millibars.

6. Process according to one of Claims 1 to 4, **characterized in that** the water containing traces cf lactams is recycled after compressing the distilled water at a pressure at most equal to the pressure of the first column and condensing at a temperature greater than 15°C.

7. Process according to one of the preceding claims, **characterized in that** water is added to the water containing traces of lactam, which is distilled in the second column, before it is recycled into the first column.

8. Process according to one of claims 1 to 7, **characterized in that** the first distillation column and the second distillation column each have at least two theoretical trays, and preferably from 2 to 10 theoretical trays.

9. Process according to one of Claims 1 to 8, **characterized in that** the distillation columns are packed columns.

## Patentansprüche

1. Verfahren zur Abtrennung von Wasser aus einer wässrigen Laktamlösung,
**dadurch gekennzeichnet, dass**:
- man das Wasser in einer ersten Destillationskolonne, indem man eine Temperatur am Fuß niedriger oder gleich 160 °C aufrechterhält, und unter einem absoluten Druck von 50 Millibar bis 200 Millibar destilliert, wobei das Destillat bei einer Temperatur von 30 °C bis 60 °C mit einem Kühlmittel kondensiert wird,
- mit dem am Fuß verbleibenden Laktam sodann in einer zweiten Destillationskolonne eine Destillation unter Auftrechterhalten einer Temperatur am Fuß niedriger oder gleich 160 °C und unter einem Druck von 10 Millibar bis 45 Millibar durchgeführt wird,
- das Laktamspuren enthaltende Wasser, das in der zweiten Kolonne destilliert wird, in Form von Dampf bei einer Temperatur höher oder gleich 70 °C gewonnen und in die erste Säule zurückgeführt wird.

2. Verfahren nach Anspruch 1, **dadurch gekennzeichnet, dass** man am Fuß der ersten Kolonne eine Temperatur niedriger oder gleich 145 °C aufrechterhält.

3. Verfahren nach einem der Ansprüche 1 oder 2, **dadurch gekennzeichnet, dass** man am Fuß der zweiten Kolonne .eine Temperatur niedriger oder gleich 145 °C aufrechterhält.

4. Verfahren nach einem der Ansprüche 1 bis 3, **dadurch gekennzeichnet, dass** das Laktam Caprolaktam ist.

5. Verfahren nach einem der Ansprüche 1 bis 4, **dadurch gekennzeichnet, dass** die Rückführung des Wassers nach einer Kondensation bei einer Temperatur von 5 °C bis 30 °C unter einem Druck von 10 bis 45 Millibar durchgeführt wird.

6. Verfahren nach einem der Ansprüche 1 bis 4, **dadurch gekennzeichnet, dass** die Rückführung des Laktamspuren enthaltenden Wassers nach Verdichtung des destillierten Wassers bei einem Druck, der höchstens gleich dem Druck der ersten Kolonne ist, und Kondensation bei einer Temperatur höher 15 °C durchgeführt wird.

7. Verfahren nach einem der vorstehenden Ansprüche, **dadurch gekennzeichnet, dass** dem Laktamspuren enthaltenden Wasser, das in der zweiten Kolonne destilliert wird, vor der Rückführung in die erste Kolonne Wasser zugegeben wird.

8. Verfahren nach einem der Ansprüche 1 bis 7, **dadurch gekennzeichnet, dass** die erste Destillationskolonne und die zweite Destillationskolonne jeweils wenigstens zwei theoretische Böden und bevorzugt 2 bis 10 theoretische Böden aufweisen.

9. Verfahren nach einem der Ansprüche 1 bis 8, **dadurch gekennzeichnet, dass** die Destillationskolonnen Füllkörperkolonnen sind.
